# FASCICULE DE BREVET EUROPEEN

(11) **EP 0 866 417 B1**
(45) Date de publication et mention de la délivrance du brevet: **09.07.2003**
(21) Numéro de dépôt: 98400656.9
(22) Date de dépôt: 20.03.1998
(51) Int. Cl.: G06K 9/00

(54) **Lecteur d'empreintes digitales sécurisé**
Sicherer Fingerabdruckleser
Secure fingerprint reader

(30) Priorité: 21.03.1997 FR 9703482
(43) Date de publication de la demande: 23.09.1998
(73) Titulaire: SAGEM SA, 75116 Paris (FR)
(72) Inventeur: Euverte, Jean-Michel, 95490 Vaureal (FR); Hallibert, Pascal, 75013 Paris (FR)
(74) Mandataire: Bloch, Gérard

(56) Documents cités:
- EP-A- 0 098 222
- EP-A- 0 194 783
- EP-A- 0 359 554
- EP-A- 0 640 933
- FAINZILBERG L ET AL: "Computer analysis and recognition of cognitive phase space electro-cardio graphic image" COMPUTER ANALYSIS OF IMAGES AND PATTERNS. 6TH INTERNATIONAL CONFERENCE, CAIP'95. PROCEEDINGS, PROCEEDINGS OF 6TH INTERNATIONAL CONFERENCE ON COMPUTER ANALYSIS OF IMAGES AND PATTERNS, PRAGUE, CZECH REPUBLIC, 6-8 SEPT. 1995, ISBN 3-540-60268-2, 1995, BERLIN, GERMANY, SPRINGER-VERLAG, GERMANY, pages 668-673, XP002045310

## Description

Un lecteur d'empreinte digitale, en fait des sillons à la surface d'un doigt formant des creux et des bosses qu'on appellera donc par la suite empreinte digitale, sert à identifier une personne par comparaison de son empreinte digitale aux empreintes d'une bibliothèque préalablement constituée.

Il est cependant possible de leurrer le lecteur, car il reconnaît une personne à travers son doigt, et on peut donc lui présenter par exemple un moulage du doigt d'une personne dont il a les empreintes en bibliothèque, ou même encore un doigt coupé de cette personne, bref l'empreinte d'un corps sans vie.

Comme le lecteur sert en général à contrôler l'accès à des informations confidentielles ou à des biens précieux, il importe donc de se prémunir contre ce genre de piratage.

La présente invention vise à augmenter la résistance du lecteur à l'égard des tentatives frauduleuses évoquées ci-dessus.

Par le document EP-A-0640 933, on connaît un lecteur d'empreinte digitale comportant en outre des moyens de détection oxymétrique. Par ailleurs, EP-A-0 098 222 enseigne la détection pléthysmographique.

Dans ces conditions, l'invention concerne un lecteur d'empreinte digitale selon la revendication 1.

Ainsi l'invention apporte une solution satisfaisante au problème posé, avec un usage intéressant de la détection photopléthysmographique pour l'authentification du doigt.

Avantageusement, il est prévu des moyens émetteurs et des moyens récepteurs de lumière, à plusieurs longueurs d'onde, agencés pour respectivement illuminer le doigt et fournir un spectre de couleur de la lumière renvoyée par celui-ci à des moyens de comparaison agencés pour authentifier le doigt par comparaison de son spectre de colorimétrie à des spectres de référence.

On peut ainsi confirmer, dans chaque cas, qu'il s'agit d'un doigt vivant.

L'invention sera mieux comprise à l'aide de la description suivante d'un lecteur d'empreinte digitale selon l'invention, en référence aux dessins annexés, sur lesquels :
- la figure 1 représente un ensemble de détection par photoplétysmographie des pulsations cardiaques perçues dans un doigt;
- la figure 2 représente schématiquement un ensemble de mesure du spectre colorimétrique du doigt;
- la figure 3 illustre le résultat des mesures ci-dessus, et
- la figure 4 illustre très schématiquement le fonctionnement d'ensemble du lecteur.

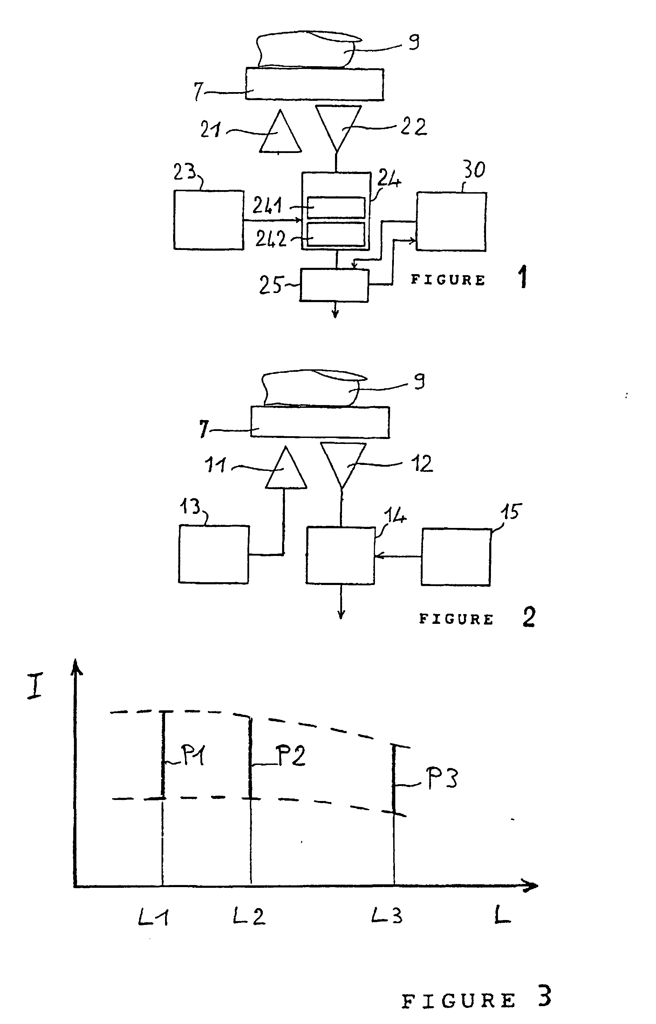

La figure 1 représente schématiquement un ensemble de détection des pulsations cardiaques perceptibles dans un doigt vivant par photoplétysmographie, c'est-à-dire détection des augmentations de volume des vaisseaux sanguins dues aux augmentations de pression liées au battement cardiaque.

Cet ensemble comporte un émetteur de lumière 21 et un récepteur de lumière associé 22 disposés contre la face du bloc 7 opposée à celle recevant le doigt 9. Le récepteur 22 alimente un circuit 24 de traitement du signal qui détermine, par une suite mesures, l'amplitude et la fréquence des variations du signal optique renvoyé par le doigt 9.

A cet effet, un circuit 241 de mesure d'amplitude détermine la valeur crête d'impulsions cardiaques par rapport à la valeur de repos séparant ces impulsions. De même, un circuit fréquencemètre ou périodemètre 242 mesure la période de répétition de ces impulsions pour déterminer la fréquence cardiaque correspondante. Une base de temps 23 fournit les signaux d'horloge nécessaires.

Ainsi, par photoplétysmographie, on vérifie que le doigt 9 est vivant et on détermine les caractéristiques cardiaques de la personne contrôlée : rythme des pulsations et amplitude.

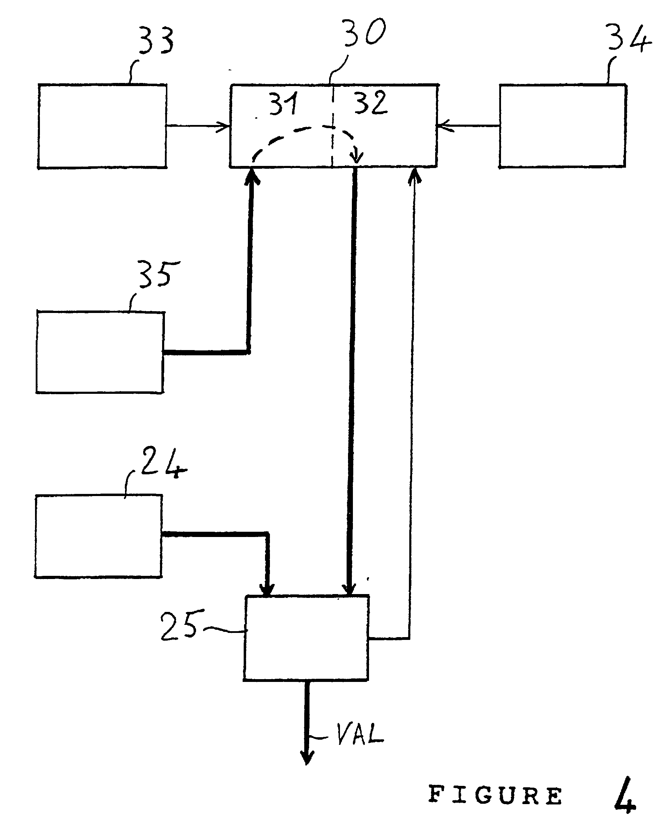

Une bibliothèque 30 contient un historique comportant, pour chaque personne autorisée, son empreinte associée à ses caractéristiques photoplétysmographiques. Cette bibliothèque 30 est adressée (figure 4) par l'ensemble classique 35 de reconnaissance d'empreinte et fournit l'identité de la personne contrôlée, si elle est ainsi reconnue. De plus, la bibliothèque 30 fournit, à un circuit comparateur 25 alimenté par le circuit de traitement 24, les caractéristiques photoplétysmographiques associées à l'empreinte. Ici, il est prévu de fournir des plages de tolérance de ces caractéristiques. Le circuit 25 compare alors les caractéristiques mesurées à celles fournies par la bibliothèque 30 qu'il consulte pour authentifier ou non la personne contrôlée. Il aurait pu être prévu que ce soit le circuit de traitement 24 qui adresse la bibliothèque 30 pour en sélectionner un groupe d'identités de personnes, dans lequel l'ensemble de reconnaissance d'empreintes 35 doit rechercher l'empreinte de Ia personne contrôlée.

Dans cet exemple, les plages de tolérance des caractéristiques sont définies de façon adaptative, c'est-à-dire que le circuit 25 fournit en retour à la bibliothèque 30 les valeurs des caractéristiques mesurées, s'il les a validées, afin de constituer, pour chaque personne, un historique de ses caractéristiques. Partant de plages de tolérance initiales, à la mise en service, relativement étendues, l'historique ou histogramme, permet de réduire et ainsi personnaliser progressivement ces plages à mesure qu'il s'enrichit et améliore ainsi l'efficacité du contrôle.

Afin de limiter le volume de matériel, il est ici en fait prévu une source lumineuse et un capteur optique, multifonctionnels, de colorimétrie et aussi de photoplétysmographie. Dans ce même but, la source lumineuse et le capteur appartiennent à l'ensemble de reconnaissance d'empreintes 35.

Sur la figure 2 est représenté schématiquement un ensemble de mesure du spectre colorimétrique d'un doigt 9 présenté et d'authentification de celui-ci, comportant une cellule comprenant un émetteur optique 11 et un récepteur optique associé 12. Une base de temps 13 assure le séquencement des divers circuits et en particulier commande l'émetteur 11, tandis que le récepteur 12 est relié à une entrée d'un comparateur 14 dont une autre entrée est reliée à une mémoire 15 contenant des spectres d'échos de doigts préalablement authentifiés et associés chacun à une identité de personne. La sortie du comparateur 14 fournit, ou non, un signal d'authentification du doigt 9 à présenter au lecteur et posé sur une face de lecture d'un bloc optique 7 servant, de façon classique, à illuminer le doigt 9 et à en saisir l'image des reliefs, ou empreinte, par des moyens non représentés. La cellule 11, 12 est accolée à la face du bloc optique 7 opposée à celle de lecture.

L'émetteur 11 est à plusieurs longueurs d'onde, ici de la lumière blanche, et le récepteur 12 détecte plusieurs longueurs d'onde, ici trois.

La figure 3 illustre le résultat des mesures, c'est-à-dire le spectre de l'intensité optique détectée I en fonction de la longueur d'onde L .

Pour chaque longueur d'onde L1, L2, L3, la mémoire 15 comporte une plage P1, P2, P3 d'intensités acceptables, définissant le spectre colorimétrique du doigt 9 et ses variations acceptables. Les deux lignes en pointillés délimitent une pluralité de plages, ou spectre, pour une bande de fréquences. Un doigt est alors authentifié si les trois mesures d'intensité sont dans la plage prévue pour les trois longueurs d'onde respectives.

La figure 4 illustre le fonctionnement global du lecteur. Par souci de clarté, l'ensemble de mesure de la figure 2 n'y a pas été représenté. La bibliothèque 30 est constituée de deux sous-ensembles 31 et 32, respectivement d'empreintes et des caractéristiques biologiques associées de chaque personne autorisée.

Pour constituer initialement la bibliothèque 30, l'ensemble 35 lit plusieurs fois l'empreinte digitale des diverses personnes autorisées, sous la commande d'un circuit 33 y associant leur identité. Une base de données 34, comportant les caractéristiques biologiques de l'espèce humaine, sert à alimenter le sous-ensemble 32.

En exploitation, l'ensemble 35 fournit une empreinte de doigt présentée au sous-ensemble 31 et ce dernier fournit, le cas échéant, l'identité de la personne reconnue. Cette identité permet d'accéder aux caractéristiques biologiques (32) de cette personne, caractéristiques qui sont initialement identiques pour toutes les personnes mais qui sont personnalisées par la suite, comme cela a été expliqué précédemment.

Le sous-ensemble 32 fournit alors ces caractéristiques biologiques au circuit comparateur 25 alimenté aussi par le circuit de lecture 24 et valide (VAL) ou non la reconnaissance de l'identité de la personne, préalablement reconnue par son empreinte. Comme indiqué, le circuit 25 fournit en retour, au sous-ensemble 32, la caractéristique mesurée et acceptée, pour constituer l'historique. L'absence de toute réponse du sous-ensemble 31 invalide donc le circuit 25.

On conçoit que chacun des deux ensembles de mesure des figures 1 et 2 suffit en lui-même pour authentifier un doigt en tant que tel et/ou pré-sélectionner une personne parmi plusieurs, et ainsi atteindre le but visé. La combinaison de ces ensembles a pour effet d'accroître l'efficacité de filtrage entre personnes, donc affecte son degré et non la nature du résultat.

## Revendications

1. Lecteur d'empreinte digitale, dans lequel il est prévu des moyens d'émission de lumière (21) et des moyens de réception de lumière (22) associés à des moyens de traitement de signal (24), agencés pour effectuer une suite de mesures de la lumière renvoyée par un doigt (9) à présenter au lecteur, pour déterminer des caractéristiques de rythme et d'amplitude de ses pulsations et pour en détecter par photoplétysmographie des pulsations cardiaques et ainsi vérifier qu'il s'agit d'un doigt (9) authentique, les moyens de traitement (24, 25) étant agencés pour consulter un historique (30) d'au moins l'une des caractéristiques d'un doigt (9) et pour authentifier ce doigt par comparaison entre la mesure et l'historique.

2. Lecteur selon la revendication 1, dans lequel les moyens de traitement (24, 25) sont agencés pour constituer l'historique (30).

3. Lecteur selon l'une des revendications 1 et 2, dans lequel il est prévu des moyens émetteurs (11) et des moyens récepteurs (12) de lumière, à plusieurs longueurs d'onde, agencés pour respectivement illuminer le doigt (9) et fournir un spectre de couleur de la lumière renvoyée par celui-ci à des moyens de comparaison (14) agencés pour authentifier le doigt (9) par comparaison de son spectre de colorimétrie à des spectres de référence (15).

4. Lecteur selon l'une des revendications 1 à 3, dans lequel il est prévu une source lumineuse et un capteur optique communs de colorimétrie et de photoplétysmographie.

5. Lecteur selon la revendication 4, dans lequel la source lumineuse et le capteur appartiennent à un ensemble de reconnaissance d'empreintes.

## Patentansprüche

1. Fingerabdruckleser, in welchem Lichtsendemittel (21) und Lichtempfangsmittel (22) vorgesehen sind, die Signalverarbeitungsmitteln (24) zugeordnet sind, die angelegt sind, um eine Reihe von Messungen des von dem Finger (9) zurückgestrahlten Lichtes vorzunehmen, welcher dem Leser vorgestellt werden soll, um die Eigenschaften des Rhythmus und der Amplitude seiner Pulsierung zu bestimmen und um durch Photopletysmographie das Herzklopfen festzustellen und somit zu prüfen, ob es sich um einen echten Finger (9) handelt, wobei die Verarbeitungsmittel (24, 25) angelegt sind, um eine geschichtliche Aufzeichnung (30) mindestens einer der Eigenschaften eines Fingers (9) zu befragen und um diesen Finger durch den Vergleich zwischen der Messungen und der geschichtlichen Aufzeichnung zu authentifizieren.

2. Leser nach Anspruch 1, in dem die Verarbeitungsmittel (24, 25) angelegt sind, um die geschichtliche Aufzeichnung (30) aufzubauen.

3. Leser nach Anspruch 1 oder 2, in welchem Sendemittel (11) und Empfangsmittel (12) für Licht mit verschiedenen Wellenlängen vorgesehen sind, die angelegt sind, um den Finger (9) jeweils zu beleuchten und ein Farbspektrum des von ihm reflektierten Lichtes an Vergleichsmittel (14) zu liefern, um den Finger (9) durch den Vergleich zwischen seinem Farbmessungsspektrum und Referenzspektren (15) zu authentifizieren.

4. Leser nach einem der Ansprüche 1 bis 3, in welchem eine Lichtquelle und ein optischer Sensor gemeinsam für die Farbmessung und Photopletysmographie vorgesehen sind.

5. Leser nach Anspruch 4, in welchem die Lichtquelle und der Sensor zu einer Abdruckserkennungsbaugruppe gehören.

## Claims

1. A fingerprint reader, wherein light emitting means (21) and light receiving means (22) associated with signal processing means (24), configured for performing a series of measurements of the light reflected by a finger (9) presented to the reader for determining rhythm and amplitude characteristics of its pulsations and for detecting cardiac pulsations by using photoplethysmography and thus verifying that it is an authentic finger (9), the processing means (24, 25) being configured for consulting a record (30) of at least one of the characteristics of a finger (9) and for authenticating said finger by comparison between the measurement and the record.

2. The reader according to Claim 1, wherein the processing means (24, 25) are configured for constituting the record (30).

3. The reader according to Claims 1 and 2, wherein light emitting means (11) and light receiving means (12) are provided having several wavelengths, configured for respectively illuminating the finger (9) and for providing a color spectrum of light reflected by the latter to a comparing means (14) configured for authenticating the finger (9) by comparison of its colorimetric spectrum to the reference spectra (15).

4. The reader according to one of Claims 1 to 3, wherein a common light source and optical sensor for colorimetry and photoplethysmography are provided.

5. The reader according to Claim 4, wherein the light source and the sensor are part of a print recognizing assembly.
